(19) **Europäisches Patentamt** / **European Patent Office** / **Office européen des brevets**

(11) **EP 4 436 474 B1**

(12) **EUROPEAN PATENT SPECIFICATION**

(45) Date of publication and mention
of the grant of the patent:
**04.02.2026  Bulletin 2026/06**

(21) Application number: **22818344.8**

(22) Date of filing: **17.11.2022**

(51) International Patent Classification (IPC):
*A61B 5/11* (2006.01)        *A46B 15/00* (2006.01)
*A61C 17/22* (2006.01)       *A46B 13/02* (2006.01)
*A61B 5/00* (2006.01)        *G16H 40/40* (2018.01)
*G16H 40/63* (2018.01)       *G16H 50/20* (2018.01)
*G16H 50/70* (2018.01)

(52) Cooperative Patent Classification (CPC):
**A46B 13/02; A46B 15/0006; A61B 5/0088;
A61B 5/1111; A61C 17/221; G16H 40/40;
G16H 40/63; G16H 50/20; G16H 50/70;**
A46B 2200/1066

(86) International application number:
**PCT/EP2022/082181**

(87) International publication number:
**WO 2023/088990 (25.05.2023 Gazette 2023/21)**

(54) **PROCESSING ARRANGEMENT FOR TOOTH MOBILITY DETECTION AND ORAL CARE DEVICE WITH SAID PROCESSING ARRANGEMENT**

VERARBEITUNGSANORDNUNG ZUR ERKENNUNG DER ZAHNBEWEGLICHKEIT UND MUNDPFLEGEVORRICHTUNG MIT DIESER VERARBEITUNGSVORRICHTUNG

DISPOSITIF DE TRAITEMENT POUR DÉTECTION DE LA MOBILITÉ DES DENTS ET DISPOSITIF SOIN BUCCAL AVEC LEDIT DISPOSITIF DE TRAITEMENT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **22.11.2021   US 202163281850 P
28.04.2022   EP 22170474**

(43) Date of publication of application:
**02.10.2024  Bulletin 2024/40**

(73) Proprietor: **Koninklijke Philips N.V.
5656 AG Eindhoven (NL)**

(72) Inventors:
• **HELAOUI, Rim
5656 AG Eindhoven (NL)**
• **HIWALE, Sujitkumar
5656 AG Eindhoven (NL)**
• **GERHARDT, Lutz Christian
5656 AG Eindhoven (NL)**
• **KOOIJMAN, Gerben
5656 AG Eindhoven (NL)**
• **RMAILE, Amir Hussein
5656 AG Eindhoven (NL)**
• **BRANDÃO SILVA, Priscilla
5656 AG Eindhoven (NL)**
• **JOHNSON, Mark Thomas
5656 AG Eindhoven (NL)**

(74) Representative: **Philips Intellectual Property & Standards
High Tech Campus 52
5656 AG Eindhoven (NL)**

(56) References cited:
**CN-A- 112 107 386        US-A- 5 518 008
US-A1- 2016 220 196**

**Description**

FIELD OF THE INVENTION

The present application relates to an oral care device, particularly an oral cleaning device.

BACKGROUND OF THE INVENTION

Mobility of teeth is a valuable clinical marker within oral care.

For example, knowledge of tooth mobility (TM) is important in connection with monitoring periodontitis disease progression, bone loss as well as for monitoring periodontal or orthodontic therapy response and therapy efficacy. Increased tooth mobility is a common symptom of periodontal disease and is one of the main reasons for a visit to a dental physician (DP).

Teeth are fixed in jawbone sockets by a periodontal ligament. The periodontal ligament not only provides firm binding support to teeth but also allows slight movement of teeth during chewing. This physiological mobility enables teeth to withstand a significant force during chewing.

Normal (physiological) tooth mobility varies between different teeth and ranges for example from 40 $\mu$m (for molars) to 120 $\mu$m (for incisors). However, certain pathological conditions of the bone or periodontal ligament can adversely affect the teeth mobility. Increased tooth mobility is a gradual and irreversible process. If not corrected in time this can also lead to loss of a tooth.

For example, 50% of Americans aged 30 or older (65 million) have periodontitis. In studies, 10-20 % of periodontitis patients showed tooth mobility of some degree and at multiple tooth sites. Increased tooth mobility can also be caused by orthodontic treatment, malocclusion issues, and bruxism.

A clinical decision as to whether tooth mobility requires a treatment response is based on detected severity or degree of tooth mobility.

A dental practitioner typically checks the mobility of a tooth by applying pressure to the tooth with a probe. Clinically, TM is usually classified in different grades such as mild, moderate and severe, based on the degree of displacement responsive to the pressure (i.e. the degree of mobility). Such classification scales have been shown to be very useful for monitoring trends in tooth mobility, with these being used as predictor for disease progression. However, these existing clinical methods for TM assessment are subjective, time consuming and error prone.

Researchers have proposed other methods for TM assessment, such as use of laser beams, or the Periotest ® (a handheld device, which applies a rapid tapping motion to the tooth or implant to assess stability based on measurement of contact time). The output of the Periotest is a tooth mobility metric in the form of a graded scale, which ranges from -8 to +50 periotest values (PTV). The lower the Periotest value, the higher is the damping effect exhibited by the test object (tooth or implant), and thus the higher is the stability of the object. Another clinical device for tooth mobility assessment is the Penguin RFA implant stability monitor which measures the resonance frequency of a temporarily attached dental screw with a non-contact technique. The frequency is displayed as an ISQ-value (Implant Stability Quotient), from 1-99. The ISQ value correlates strongly to the micro mobility of the implant.

However, there is currently no home-based method for quick and objective screening of tooth/implant mobility or stability. Having such a method could be helpful for early detection of tooth mobility or to detect stability of dental implants.

Furthermore, tooth mobility assessment at present is performed at wide time intervals, through visits to a dental practitioner. Such ad hoc analysis is not sufficient to properly analyse progression of tooth mobility in different classes such as physiological versus pathological; acute versus chronic tooth mobility. A long-term assessment of tooth mobility is also important to study the stability or changing appropriateness of dental implants over time. Moreover, existing methods do not provide any predictive capability. Such prediction could be useful in scheduling future dental consultations, or to modify a treatment regime.

Thus, in summary, existing clinical methods for measurement of tooth mobility have certain disadvantages associated with them, which include the following:

- Subjectivity in assessment of tooth mobility or the stability of a dental implant.
- Non-standardization of mobility assessment, meaning that measures obtained at different times cannot be directly compared.
- Need for dedicated hardware, limiting potential application at home.
- Time consuming for a clinician. This can typically lead to the common practice of assessing tooth mobility only when the underlying gum disease is already in an advanced stage.
- No information on likely future trend of tooth mobility or dental implant's stability.

Document CN112107386A discloses a smart oral care device for acquiring and processing health data of users, including for example loose teeth.

## SUMMARY OF THE INVENTION

An important challenge in providing tooth mobility assessment is the provision of an objective, standardized metric for tooth mobility that allows for measures obtained for different teeth and at different time to be compared, and allows for clinical decisions to be made based on individual absolute measures (rather than relying on trends over time for example).

One aim of embodiments proposed herein is to provide a metric of tooth mobility (TM) which is objective and standardized and which allows to directly compare mobility of different teeth, to perform clinical assessment, and to derive a longitudinal trends of TM.

The invention is defined by the claims.

According to examples in accordance with an aspect of the invention, there is provided a processing arrangement for an oral care device, comprising:

an input/output; and
one or more processors, adapted to:

receive at the input/output a sensing signal indicative of a loading force or pressure applied to, or motion of, at least one cleaning element of the oral care device or a support body to which the cleaning elements are coupled, wherein the signal spans a time period, the time period corresponding to a period of mechanical engagement of the at least one cleaning element with at least one tooth during an oral cleaning session,
process a waveform of the signal to extract one or more characteristics of the signal waveform for the time period;
obtain a measure of at least one further characteristic, the further characteristic being the tooth type, and/or a characteristic of the mechanical engagement of the at least one cleaning element with the tooth;
determine a metric of tooth mobility for the at least one tooth in dependence the one or more signal characteristics and upon the at least one further characteristic; and
generate a data signal indicative of the determined metric, and provide the data signal to the input/output.

Thus embodiments propose to derive a metric of tooth mobility based on force, pressure or motion information related to cleaning elements in a home-based oral care device. For example, the tooth mobility can be assessed from sensor measurements acquired during an oral cleaning session of an oral cleaning device such as a toothbrush or mouthpiece device. In this way, tooth mobility can be assessed at home, in a convenient manner.

In addition to the cleaning element motion characteristic data, embodiments propose to use at least one further characteristic related to the tooth being sensed, or related to the mechanical engagement between the at least one cleaning element and the tooth, so as to standardize or normalize the obtained metric and make it objectively comparable with metrics obtained for other teeth, and under different measurement conditions, e.g. in a subsequent or earlier cleaning session. The additional characteristic increases the specificity of the metric.

The cleaning elements may be bristles in some examples.

In some embodiments, the obtained metric of tooth mobility may define a graded level of tooth mobility.

In some embodiments, the processing arrangement may be further adapted to: access a datastore recording historical tooth mobility metric data for a user; and output the data signal to the datastore, for thereby storing the determined tooth mobility metric in the datastore.

In some embodiments, the processing arrangement may be further adapted to: access the datastore recording historical tooth mobility metric data for the user and determine a longitudinal trend in tooth mobility metrics for one or more teeth of the user based on the historical tooth mobility metric data and the determined metric of tooth mobility in the current cleaning session.

In some embodiments, the processing arrangement may be further adapted to generate a prediction of tooth mobility progression based on the longitudinal trend.

The processor may generate a data signal indicative of the longitudinal trend for output from the device. This may for example be output to a datastore (the same or a different datastore), or to a user interface.

In some embodiments, the processing arrangement may be further adapted to obtain an identification of the at least one tooth. The data signal referred to above may further include the identification of the at least one tooth.

The processor may be further adapted to obtain an indication of a tooth type of the at least one tooth. The at least one further characteristic referred to above may include the tooth type. Different teeth have different external tooth morphologies, and different root structures (e.g. one root, two roots, three roots) which affect normal tooth mobility. Classification of tooth mobility may take account of the tooth type.

The information regarding type of teeth can be obtained for example from a location sensor integrated within the oral cleaning device or it can be obtained from other methods such as camera imagery by using standard image processing methods.

In some examples, determining the metric of tooth mobility may comprise applying a machine learning algorithm. In some examples, determining the metric of tooth mobility may comprise applying a multi-parametric linear regression algorithm.

In some embodiments, the received sensing signal may comprise signal segments corresponding to respective periods of engagement of the at least one cleaning element with each of a plurality of teeth in series. The processing arrangement may be adapted to determine a respective metric of tooth mobility for each of the plurality of teeth. It may be further adapted to obtain an identification of each of the teeth.

The sensing signal covers periods of engagement of the at least one cleaning element with a plurality of teeth in series. Thus, the processing arrangement may divide the signal into a plurality of signal segments, each covering a respective period of engagement with a respective tooth.

The dividing of the signal into segments may comprise: identifying a start and end of each signal segment by: detecting a pre-determined signature pattern indicative of start and end of each tooth segment; or using a positioning signal for the at least one cleaning element, received from a positioning signal source. The dividing of the signal might in other cases be based on a user input.

With regards to the at least one further characteristic of the tooth, and/or of the mechanical engagement of the at least one cleaning element with the tooth, there are different options.

In some embodiments, the at least one further characteristic may comprise a baseline user load parameter, indicative of a baseline or average loading force exerted on the cleaning element by the user during use.

In some embodiments, the processing arrangement may be adapted to perform a calibration operation comprising determining the baseline or average loading force applied to the at least one cleaning element during a cleaning session, using the sensing signal.

The baseline or average force is representative of the user-applied load.

In some embodiments, the at least one further characteristic may include a brushing stroke speed parameter indicative of a speed of user-applied brushing strokes during the cleaning operation.

This could be determined in a calibration operation and stored. It could be determined dynamically during the cleaning sessions, for instance using an inertial measuring unit (IMU).

In some embodiments, the at least one further characteristic may comprise one or more structural characteristics of the at least one cleaning element. These may for example include one or more of: an angle of protrusion of the cleaning elements; and a metric of stiffness of the cleaning elements.

Thus, specific properties relating the cleaning elements (such as for instance type of bristle, stiffness of bristle, frictional coefficient of bristle, wear of bristles, shape of bristles, layout of bristles) can be used as the further characteristic.

In some embodiments, the processing arrangement may be further adapted to process the obtained tooth mobility metric and to derive a clinical classification of the tooth mobility for the at least one tooth. Thus, this embodiment proposes to classify the tooth mobility metric according to a clinically relevant category, allowing for clinical interpretation and cross-correlation between teeth and between cleaning sessions, to clinically assess tooth mobility, e.g. a degree of disease, or a disease prediction indicator.

The clinical classification in other words puts the tooth mobility into a clinical context, i.e. indicating the degree to which the measured level of tooth mobility is normal or abnormal, i.e. a level of pathology of the mobility.

Deriving the clinical classification may comprise comparing the obtained tooth mobility metric with a selected one or more thresholds.

In some embodiments, the one or more thresholds may be selected or set based on at least one of: a type of tooth, and a demographic classification of the user.

In some embodiments, where the sensing signal includes signal segments corresponding to periods of engagement of the at least one cleaning element with a plurality of teeth, and where the processor is adapted to determine a respective metric of tooth mobility for each of the plurality of teeth, deriving the clinical classification for the tooth mobility for a first tooth may further comprise comparing the tooth mobility metric obtained for the first tooth with a further tooth to detect a level of deviation. The further tooth is preferably of a same tooth type. It is preferably on an opposite side of the dental arch to the first tooth. The level of deviation is preferably further compared with a deviation threshold to thereby obtain the clinical classification.

In some embodiments, the processing arrangement may be further adapted to: access a datastore recording historical tooth mobility metric data for a user; and compare the obtained tooth mobility metric for the at least one tooth with historical values for the same tooth. The clinical classification may further be based on said comparison.

In an advantageous set of embodiments, the tooth mobility metric is used to derive predictive data concerning predicted progression of tooth mobility or clinical classification.

For example, in some embodiments, the processing arrangement is adapted to generate a prediction of tooth mobility progression based on application of a machine learning algorithm.

In some embodiments, the machine learning algorithm may be trained to receive as inputs: the derived tooth mobility metric for a single cleaning session, and a demographic classification of the user, and to generate as an output a prediction of tooth mobility progression, and wherein the algorithm has been trained based on training data for a population of multiple patients in different demographic classifications. The training data may comprise, for each of the multiple patients, tooth mobility metrics at two different time points, and optionally a demographic classification for the patient.

Additionally or alternatively, in some embodiments, a machine learning algorithm may be provided which is trained to receive as inputs: the derived tooth mobility metric for a series of cleaning sessions for a series of time points, and to generate as an output a prediction of tooth mobility progression, and wherein the algorithm has been trained based on training data for a population of multiple patients. The training data may comprise for each of the multiple patients tooth mobility metrics for a series of different time points.

Determining the metric of tooth mobility may involve initially determining a provisional indication of a degree of tooth mobility. This may in some examples comprise performing a spectral analysis of the sensing signal.

In particular, the one or more characteristics of the signal waveform referred to above may comprise an amplitude of a selected one or more frequency components of the signal, and/or a bandwidth associated with a dominant (i.e. resonant or harmonic) peak in the frequency spectrum of the sensing signal or a quality factor associated with a dominant peak of a frequency spectrum of the sensing signal.

In particular, the processor may be adapted to: compute a frequency spectrum for the sensing signal. It may be adapted to detect an amplitude of one or more frequency components of the sensing signal. It may be adapted to detect an amplitude of a central frequency of a dominant peak of the frequency spectrum. It may be adapted to detect a bandwidth associated with a dominant peak of the frequency spectrum, and/or a quality factor associated with a dominant peak of the frequency spectrum.

In this disclosure, a dominant peak refers to a peak having a highest amplitude at its center frequency among the peaks in the frequency spectrum. This is otherwise known as the main harmonic peak, or resonant peak.

Determining the metric of tooth mobility may comprise comparing one or more of the above-mentioned characteristics of the frequency spectrum with a reference of baseline. Alternatively, it may comprise processing one or more of the above-mentioned characteristics with a transfer function or lookup table to derive the metric of tooth mobility.

The oral care device may include an actuation means for driving an oscillatory motion of the at least one cleaning element which, in a reference condition, exhibits a reference frequency spectrum with at least one dominant (i.e. main harmonic or resonant) peak having a central frequency.

In some examples, the determining the degree of tooth mobility may comprise determining changes between the reference frequency spectrum and the sensed frequency spectrum of:

- an amplitude of the central frequency component of the aforementioned dominant peak of the frequency spectrum ;
- a bandwidth associated with the dominant peak of the frequency spectrum; and/or
- a quality factor associated with the dominant peak of the frequency spectrum.

In other words, the oscillatory motion has a spectral signature, and wherein determining the metric of tooth mobility comprises detecting changes in said spectral signature. However it is not essential to compare the measured frequency spectrum characteristics with such a reference spectrum. As noted above, in other cases a transfer function, such as a multi-parametric linear regression function could be used to derive the metric of tooth mobility from the one or more characteristics of the frequency spectrum, and the one or more further characteristics.

The reference frequency spectrum referred to above could for instance correspond to an expected motion frequency spectrum for a controlled calibration condition, for instance for a reference load, and for a certain reference tooth (e.g. a non-mobile tooth), e.g. in a brushing session after a dental examination, or against a well-defined surface using a standardized method.

The reference frequency spectrum could be stored as calibration data in a memory of the processing arrangement.

There could be stored multiple different reference frequency spectra corresponding to different possible applied user loads.

Another aspect of the invention provides an oral care device comprising:

a support body;
a plurality of cleaning elements for engagement with oral surfaces, the cleaning elements protruding from a surface of the support body;
a sensor means adapted to sense a measure indicative of a loading force applied to, or a motion of, at least one of the cleaning elements or the support body; and
a processing arrangement in accordance with any embodiment described in this disclosure, or in accordance with any claim of this application.

In one non-limiting set of examples, the oral care device is a powered toothbrush. In a further non-limiting set of examples, the oral care device is a brushing mouthpiece device.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates an example oral care device in accordance with one or more embodiments;
Fig. 2 illustrates a block diagram of components of an example oral care device in accordance with one or more embodiments;
Fig. 3 illustrates a relationship between sensing signal response and degree of tooth mobility;
Figs 4-6 illustrate simulation results indicating how a degree of tooth mobility may be derived from a spectral analysis of a sensing signal:
Fig. 7-8 illustrate simulation results indicating how applied user load affects measured sensing signal response for a fixed degree of tooth mobility;
Fig. 9 illustrates how tooth mobility and user load may be correlated;
Fig. 10 illustrates how a clinical metric may be derived from a tooth mobility metric;
Fig. 11 illustrates an example decision tree for classifying tooth mobility of teeth;
Fig. 12 illustrates the architecture and workflow of an example machine learning model;
Fig. 13 illustrates predicting future tooth mobility values; and
Fig. 14 illustrates a summary of example embodiments.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods will become better understood from the following description, and accompanying drawings. It should be understood that the Figures are merely schematic and are not drawn to scale. It should also be understood that the same reference numerals are used throughout the Figures to indicate the same or similar parts.

The invention provides a means for deriving a metric of tooth mobility based on force or motion data acquired from a sensor means integrated in an oral care device and acquired during an oral cleaning session. By processing a waveform of the sensing signal to detect one or more signal characteristics, and further acquiring one or more characteristics of the tooth-device mechanical engagement, a standardized metric of tooth mobility can be derived.

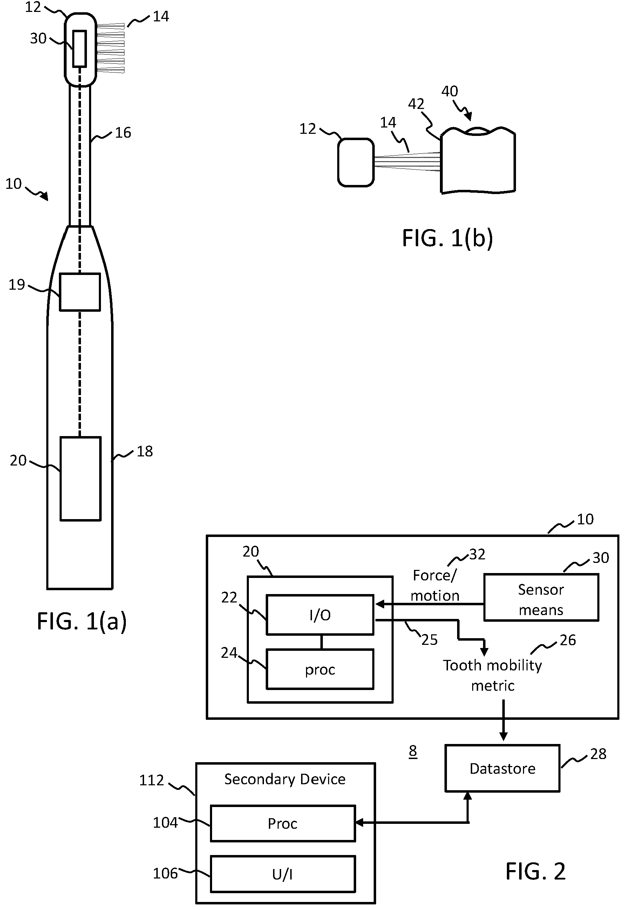

Fig. 1 and 2 illustrate an oral care device 10 which is in accordance with at least one set of embodiments of the invention. The device 10 comprises a support body 12, from at least one surface of which protrude a plurality of cleaning elements 14 for mechanical engagement with oral surfaces 42. The oral surfaces 42 may be surfaces of teeth 40 or for instance of dental implants. The device further includes a sensor means 30 adapted to sense a measure indicative of a loading force applied to, or motion of, at least one of the cleaning elements 14 or the support body 12.

The device further comprise a processing arrangement 20.

The processing arrangement comprises an input/output 22; and one or more processors 24, adapted to: receive at the input/output 22 a sensing signal 32 indicative of a loading force applied to, or motion of, at least one cleaning element 14 of the oral care device. The signal spans a time period, the time period corresponding to a period of mechanical engagement of the at least one cleaning element with at least one tooth 40 during an oral cleaning session.

The processing arrangement is adapted to process a waveform 60 of the signal to extract one or more characteristics of the signal waveform for the time period.

The processing arrangement is further adapted to obtain a measure of at least one further characteristic, the further characteristic being a characteristic of the tooth, and/or of the mechanical engagement of the at least one cleaning element with the tooth.

The processing arrangement is further adapted to determine a metric of tooth mobility 26 for the at least one tooth in dependence upon the one or more signal characteristics and upon the at least one further characteristic, and to generate a data signal 25 indicative of the determined metric, and provide the data signal to the input/output 22.

The device may further include a user interface for providing a user output indicative of tooth mobility metric.

Thus, embodiments propose a method for providing a standardized tooth mobility metric for one or more teeth, acquired during one or more cleaning sessions.

The input data include data from a sensing means which could sense a force, pressure, and/or motion signal (such as acceleration), wherein the sensing means may for instance be integrated in the support body (e.g. a brush head of a toothbrush or in a device handle).

The input data further include at least one further characteristic of the tooth, and/or of the mechanical engagement of the at least one cleaning element with the tooth. By way of non-limiting illustration, this could include brushing speed (stroke speed), brushing duration, brushing angle, or vibration frequency (or derivatives of these parameters)

In some embodiments, the input data may further include positioning information, allowing for identification of a particular tooth to which a tooth mobility metric corresponds. The positioning data could be derived from a position/orientation sensor integrated in the oral care device in some examples. The positioning data could also be derived from a motion sensor.

In some embodiments, the processing arrangement is further adapted to generate a data message for transmission to a clinician device, to provide an indication when a certain level of tooth mobility or an increase in tooth mobility is detected.

With reference to Fig. 1, the oral care device in the illustrated embodiment is shown in the form of a toothbrush with a brush head 16 coupled to a main body 18. The main body forms a handle for the device. The support body 12 is provided by a platen of the brush head. The cleaning elements 14 may comprise bristles. The bristles may be arranged into tufts. The reference to at least one cleaning element above could be reference to at least one tuft of bristles in this set of embodiments.

Although bristles are mentioned as an example, the skilled person will be aware that other varieties of cleaning element are also possible, such as elastomeric cleaning elements (thicker than a typical individual bristle), or cleaning structures which protrude from the support body having extended shapes, but which are also designed to engage with oral surfaces to provide a mechanical cleaning action.

Although a toothbrush is shown in Fig. 1, in further examples, the oral care device could take a different form, such as a brushing mouthpiece device, or an oral irrigation device, or an oral flossing device.

The block diagram structure of Fig. 2 is applicable to an oral care device of any particular type, not just to a toothbrush.

With further reference to Fig. 1, preferably the oral care device includes an actuation means 19 for driving an oscillatory motion of the at least one cleaning element 14.

This induced oscillatory motion pattern of the at least one cleaning element exhibits a frequency spectrum. The device may record in a memory (not shown) of the processing arrangement 20 one or more reference frequency spectra which correspond to frequency spectra of the driven oscillatory motion pattern in certain reference or calibration conditions, such as in the presence of a certain reference loading force and to a certain tooth with a reference level of mobility (e.g. zero mobility). The reference condition could be any desired condition, e.g. in the absence of externally applied loading forces to the at least one cleaning element, and/or for a tooth with non-zero mobility. There could be more than one reference spectrum stored, for different conditions, e.g. different applied used loads (this is discussed further below). In all cases, the reference frequency spectrum has at least one dominant peak (which corresponds effectively to the main harmonic component or main resonant component of oscillation). There may be multiple dominant peaks, for instance each at a respective harmonic of the main frequency component of the oscillation. The dominant peaks in this context means those with the highest amplitude at their central frequency. Reference to the dominant peak (singular) means the peak with the highest amplitude among all peaks in the frequency spectrum.

The dominant peak of the frequency spectrum has a bandwidth and a quality factor associated with it. By comparing characteristics of a frequency spectrum of a sensed oscillatory motion pattern with characteristics of the reference oscillatory motion pattern, a level of tooth mobility may be detectable.

The sensor means 30 referred to above may take different forms, for example a pressure sensor, force sensor, inertial sensor (e.g. accelerometer), or load cell. The sensor means may be adapted to sense variations of acceleration, pressure, and/or force in the support body or the cleaning elements in one or more directions, e.g. normal to the support body surface from which the cleaning elements protrude, or preferably, in the three orthogonal axes.

The processing arrangement is adapted to convert the sensing loading signal variations into a tooth mobility level or grade identification.

With reference to Fig. 2, optionally in some embodiments, the processing arrangement 20 is further adapted to: access a datastore 28 recording historical tooth mobility metric data for a user; and output the data signal 25 to the datastore, for thereby storing the determined tooth mobility metric in the datastore.

In some embodiments, the one or more processors 24 are adapted to: access the datastore 28 recording historical tooth mobility metric data for the user and determine a longitudinal trend in the tooth mobility metric for one or more teeth of the user based on the historical tooth mobility metric data and the determined metric of tooth mobility for the current cleaning session. The one or more processors may be further adapted to generate a prediction of tooth mobility progression based on the longitudinal trend.

The processor may generate a data signal indicative of the longitudinal trend for output from the device. This may for example be output to a datastore (same or different datastore), or to a user interface.

An identification of the particular tooth to which each mobility metric corresponds may also be obtained (for instance using a positioning means), and this identification stored in association with the tooth mobility metric in the data record.

With reference to Fig. 2, another aspect of the invention may provide a system 8 comprising: an oral care device 10 in accordance with any embodiment described in this disclosure or in accordance with any claim of this application; and further comprising a datastore 28 for storing previously determined metrics of tooth mobility for one or more subjects.

With reference again to Fig. 2, optionally, in some embodiments, the system may further comprise a secondary device 112 comprising a processing arrangement 104 adapted to access the datastore 28 and to retrieve multiple tooth mobility data records for a given subject for one or more different time points.

Data records for a plurality of different time points may be retrieved, each record comprising tooth mobility metrics for one or more teeth. The processor 104 of the secondary device 112 may generate by processing the retrieved data records trend information indicative of the one or more tooth mobility metrics as a function of time. The processor 104 of the secondary device 112 may control a user interface 106 of the secondary device to display the trend information.

The secondary device may for example comprise a mobile computing device such as a smartphone.

Another aspect of the invention provides the processing arrangement 20 alone, without the other components of the oral care device 10 or of the system 8.

Deriving the metric of tooth mobility is a process based on two main parts. A first stage is deriving from the sensing signal waveform a measure indicative of a relative level of tooth mobility (i.e. which is correlated with tooth mobility level). Second stage may be converting the relative level of tooth mobility into an objective metric based on the further use of at least one further characteristic related to the tooth or to the mechanical engagement of the at least one cleaning element with the tooth. Alternatively, these two parts may be implemented by a single algorithm or function in some examples, e.g. using a multiparametric regression function.

These two parts of the processing operation will now be each discussed further.

As mentioned, a first requirement is to obtain a measure indicative of a level of tooth mobility, where this may be a relative level, i.e. non-standardized, and therefore not directly comparable between teeth or between cleaning sessions. In other words, it is a measure which is correlated with tooth mobility, but does not necessarily given an objective absolute measure.

For this purpose, in some embodiments, the processing arrangement may be adapted to compare the one or more characteristics of the sensing signal waveform with corresponding characteristics for a stored baseline or reference sensing signal to determine the tooth mobility level. The reference signal may for example correspond to a signal for a certain reference tooth (e.g. permanent healthy tooth which could be indicated by a dental practitioner).

In some embodiments, an averaged sensing signal for multiple cleaning sessions may be computed by storing the sensing signal for each session in a memory and then retrieving the stored signals and computing the average. This average signal could be used for determining the level of tooth mobility instead of (or in addition to) the signal for just a single cleaning session.

In some embodiments, the measure of tooth mobility could be derived for a given tooth by comparing the one or more characteristics of the signal waveform for the given tooth with those of one or more other teeth acquired within the same cleaning session.

In general terms, the sensing means 30 incorporated in the oral cleaning device can be used to record applied pressure (user load) and resistance from a tooth by analyzing reaction forces (or pressures/accelerations) on the support body 12. This information can be used to derive tooth mobility by analyzing the variation of characteristic parameters, such as oscillation amplitude at one or more frequency components, or damping of oscillation, derived from normal and/or tangential reaction forces, pressures, or accelerations exerted on the support body and/or the cleaning elements 14.

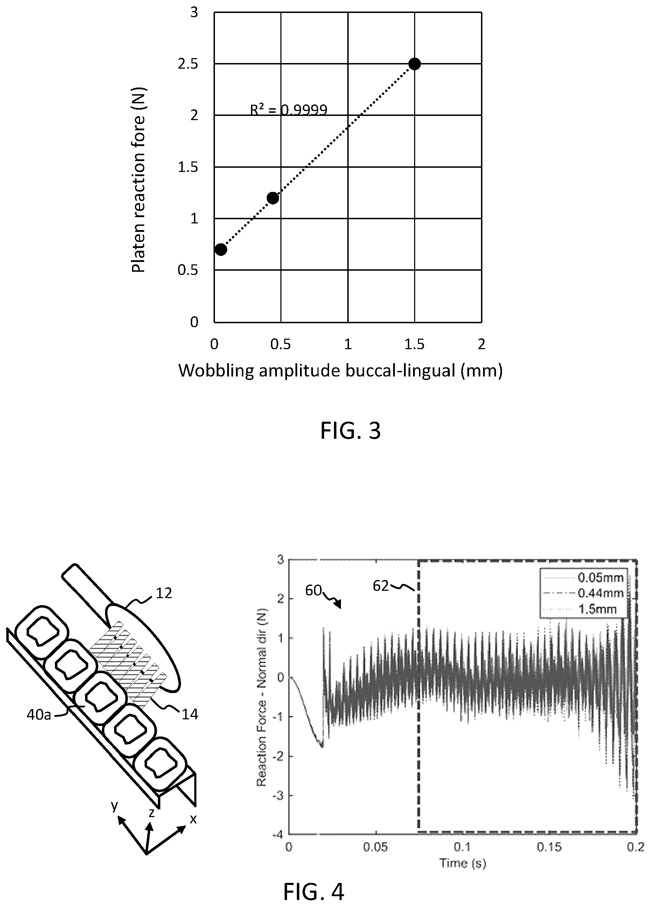

For example, Fig. 3 illustrates a derived (linear) relationship between tooth mobility displacement amplitude (wobbling amplitude) and corresponding support body normal reaction force (bouncing back of tooth impinging the cleaning elements and therefore the support body). The forces are relatively large, e.g. in the illustrated example, from 0.7-2.5 N, and this could be measured for example using a Hall sensor.

In at least one set of embodiments, it is proposed to determine tooth mobility using a spectral analysis of the sensing signal. For example, determining tooth mobility may comprise assessing, within the frequency domain of the sensing signal, variations of signal amplitude at one or more frequency points, and/or damping of a main harmonic component of the oscillation pattern over one or more cleaning sessions. The variations may be monitored over multiple cleaning sessions, or within the same cleaning session using differential measurement among different teeth.

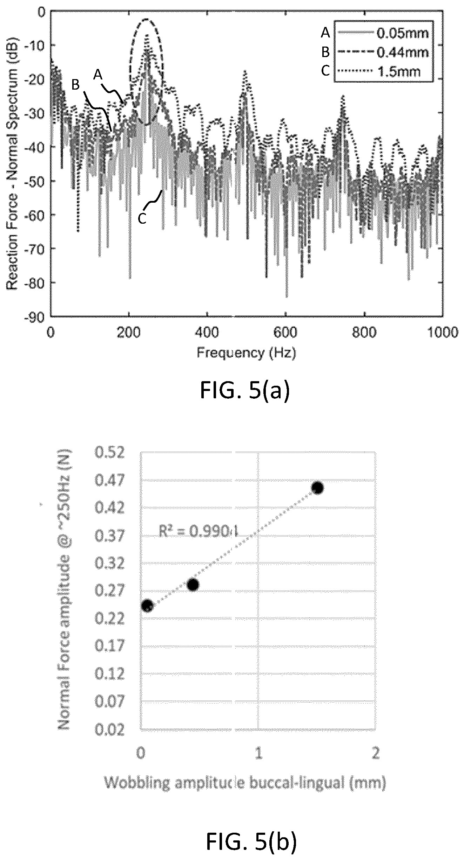

With reference to Fig. 4 and Fig. 5, the sensing principle is illustrated. Fig. 4 shows mechanical engagement of cleaning elements 14 of an example oral care device 10 with teeth 40. The user, in cleaning their teeth, applies the cleaning elements with a certain user load force, which includes a component normal to the surface of the tooth. As illustrated in Fig.

4, when the cleaning elements engage a tooth which has a high level of mobility ("wobbly tooth"), the tooth exhibits motion. The motion of the highlighted tooth 40a is predominantly motion about the y-axis, but also exhibits some smaller degree of motion about the x-axis.

With reference to Figs. 4-6, computer simulations were run to test and demonstrate the feasibility of detecting tooth mobility. The model employed an *in-silico* tooth-jaw model.

Figs. 4-7 relate to a simulation in which three different levels of tooth mobility of a tooth of interest 40a were tested: low tooth mobility (the maximum tooth displacement being 0.05mm, representative of normal physiological tooth movement); medium tooth mobility (maximum tooth displacement of 0.44 mm) and high level of tooth mobility (the maximum tooth displacement being 1.5 mm, typical of tooth mobility for a tooth that will subsequently be lost).

The in-silico model consisted of a jaw with soft gums around the reference tooth 40a, and wherein the reference tooth is free to rotate around the mesial-distal (Y) axis and is connected through a torsional spring to the root.

The level of mobility of the reference tooth is controlled in the model by tuning the stiffness coefficient of the torsional spring attached to it. Brushing conditions were simulated based on a Philips A3 brush head, with a 90° angle of the cleaning elements relative to the tooth surface (90° brushing angle) and a constant user load of 1.88N (average load among Philips toothbrush users). The motion pattern of the cleaning elements was modeled as a large-amplitude medial-distal stroke (applied by a user) superposed with a smaller amplitude oscillatory motion pattern induced by the actuation/drive mechanism of the powered toothbrush.

A simulated sensing signal representing reaction forces on the brush head platen 12 as a function of time, as well as the simulated tooth displacement, were computed from the model for the three different mobility conditions mentioned above.

Fig. 4 (right) shows a representation of a waveform 60 of the obtained simulated sensing signal for the three different mobility conditions in the time domain.

The received sensing signal waveform 60 may typically comprise signal segments corresponding to respective periods of engagement of the at least one cleaning element with each of a plurality of teeth. The processing arrangement 20 may be adapted to determine a respective metric of tooth mobility for each of the plurality of teeth. An identification of each of the teeth may further be obtained in some examples.

Within the simulated sensing signal result of Fig. 4, a signal segment 62 corresponding to the modelled tooth of interest 40a is indicated.

In accordance with at least one set of embodiments, determining the degree of tooth mobility may comprise performing a spectral analysis of the sensing signal.

This is illustrated further with reference to Fig. 5(a).

To compute the measure indicative of a degree of tooth mobility, the processing arrangement in accordance with at least one set of embodiment is adapted to compute a frequency spectrum 70, 72 for the sensing signal. This can be done for example with a Fourier Transform, e.g. a Fast Fourier Transform (FFT).

The plots of Fig. 5(a) each show frequency spectra for each of the three different tooth mobility levels, labelled A, B and C. Level A corresponds to a mobility amplitude of 0.05 mm. Level B corresponds to a mobility amplitude of 0.44 mm. Level C corresponds to a mobility amplitude of 1.5 mm. Fig. 5(a) shows the frequency spectra 70 computed for the normal reaction force components (x-direction in Fig. ) of the sensing signal at each of the three different tooth mobility levels, A, B, C.

As mentioned above, in this example, the oral care device is one which includes an actuation means 19 for driving an oscillatory motion of the at least one cleaning element 14 which exhibits a baseline/reference oscillatory frequency spectrum with a central frequency component.

Observing the frequency domain plots 72, 74 for the reaction forces, there is a large increase in the reaction force amplitude levels for frequency components around the harmonics of the dominant frequency (around 250 Hz) of the reference frequency spectrum of the actuation means. There is also an increase in the damping level between frequency components around these harmonic frequencies. In particular, the amplitude of the frequency component corresponding to the main harmonic component of the oscillatory driving force applied by the actuation means (at around 250 Hz) shows an amplitude increase of about 4dB (in the normal direction) and 9.6dB (in the mesial-distal direction) from the lowest to the highest level of tooth mobility simulated.

The changes in the damping around the driving oscillation harmonics are qualitatively observable by the changes in bandwidths, and could be determined quantitatively by, for instance, computing the quality factor (Q factor), i.e. a dimensionless parameter defined as the ratio of the resonance center frequency to its bandwidth. By resonance center frequency is meant the central frequency component of the dominant peak in the frequency spectrum. For example, for a -3dB bandwidth, the Q factor for the dominant peak (main harmonic peak) may range from 30 in the case of a low level of mobility (case A) to 25 a high mobility case (case C) for the normal force, and, similarly for the mesial-distal force, it reduces from 31 to 25.7.

Fig. 5(b) shows a resulting derived relationship between amplitude of the normal reaction force at the central frequency of the dominant peak of the frequency spectrum of the actuation means (e.g. 250 Hz in this particular example), and the wobbling amplitude of the tooth of interest (i.e. the level of tooth mobility). The reaction force is measured by the sensing means 30 in the oral care device.

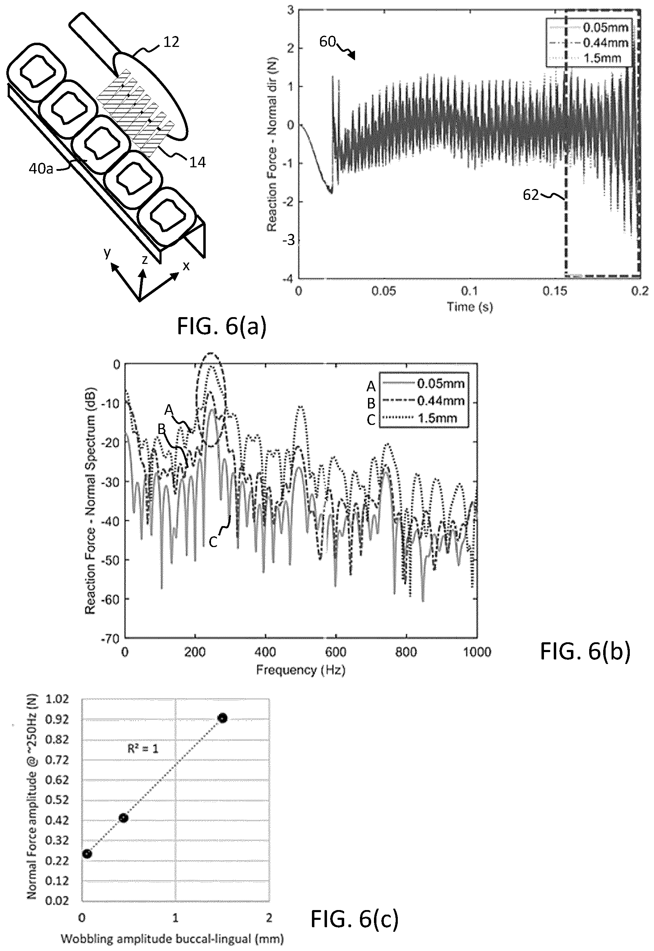

Fig. 6 illustrates a computed relationship between the level of tooth mobility and normal reaction force amplitude, for a different signal segment 62 spanning a different time period.

Fig. 6(a) shows the same simulated time-domain sensing signal as in Fig. 4.

Fig. 6(b) shows the computed frequency spectra for the three different tooth mobility levels A, B, C over a smaller temporal signal segment 62. The signal segment 62 of Fig. 6 corresponds to a time window of 0.075s - 0.2s. The signal segment of Fig. 5 corresponds to a time window of 0.16s-0.2s.

Fig. 6(c) shows a resulting derived relationship between amplitude of the normal reaction force at the central oscillation frequency of the actuation means (e.g. 250 Hz in this particular example), and the wobbling amplitude of the tooth of interest (i.e. the level of tooth mobility. The reaction force is measured by the sensing means 30 in the oral care device. Thus, it can be seen that different levels of tooth mobility can be related to different measured force levels no matter the time window chosen to analyze the measured signals.

Thus, in summary, following this approach, in accordance with at least one set of embodiments, determining the degree of tooth mobility may comprise detecting an amplitude of one or more frequency components of the sensing signal, and more particularly based on changes in an amplitude of a central frequency of a dominant peak in the oscillation frequency spectrum.

In some examples, determining the degree of tooth mobility may be performed by determining changes between the frequency spectrum of the oscillation applied by the actuation means and the sensed frequency spectrum.

Additionally or alternatively, in some examples, determining the degree of tooth mobility may be performed by determining changes between the oscillatory frequency spectrum of the oscillation applied by the actuation means and the sensed frequency spectrum of: a bandwidth associated with a dominant peak of the frequency spectrum or a quality factor associated with a dominant peak of the frequency spectrum.

Based on these results, the measured normal reaction force amplitude at a central frequency of a dominant (or main harmonic) peak of the oscillation frequency spectrum (referred to herein as: Fn), and level of tooth mobility (TM), can be seen to be directly proportional. Thus the reaction force amplitude at the dominant frequency can be used as a straightforward proxy parameter for tooth mobility (TM). It can be expressed in a mathematical form as:

$$\text{Reaction force amplitude (Fn)} = f(\text{TM}) \qquad \ldots (1)$$

where TM represents a level of tooth mobility.

In some embodiments, the processing arrangement may be further adapted to obtain an identification of the at least one tooth, and wherein the data signal further includes the identification of the at least one tooth.

The identification may be obtained using positioning data for the at least one cleaning element. This could for example be obtained using an orientation sensor or motion sensor integrated in the oral care device (e.g. an inertial measurement unit (IMU)).

Additionally or alternatively, tooth identification can be derived by analysis of the sensing signals.

For example, as previously discussed, the received sensing signal may comprise signal segments 62 corresponding to respective periods of engagement of the at least one cleaning element with each of a plurality of teeth, and the processing arrangement adapted to determine a respective metric of tooth mobility for each of the plurality of teeth, and obtain an identification of each of the teeth.

A start and end of each signal segment 62 can be identified by: detecting a predetermined signature pattern indicative of start and end of each tooth segment; or using of a positioning signal for the at least one cleaning element, received from a positioning signal source. In this way, it can be identified which portions of a sensing signal relate to different teeth.

The processor may in some examples be further adapted to identify a tooth type to which each signal segment corresponds based on a user input indicative of the tooth type.

As discussed above, deriving the tooth mobility metric comprises further obtaining a measure of at least one further characteristic, the further characteristic being a characteristic of the tooth, and/or of the mechanical engagement of the at least one cleaning element with the tooth.

Different factors such as cleaning pressure (user load), angle of cleaning elements relative to teeth, properties of a tooth being brushed, material properties of cleaning elements and geometrical layout of the cleaning elements are likely to impact the measured dynamic sensing signal (e.g. reaction force). This makes it difficult to directly compare characteristic parameter values obtained from different teeth or from different brushing sessions, without taking such additional factors into account in determining a tooth mobility metric.

The inventors have studied, using simulation experiments, the above-mentioned factors to determine their respective impact on the sensing signal response. For each simulation experiment, one parameter was changed at a time while keeping other parameters constant.

The different options for the one or more further characteristics used in determining the tooth mobility metric will now be discussed.

In accordance with at least one set of embodiments, the at least one further characteristic comprises a user baseline load parameter, indicative of a baseline or average loading force exerted on the cleaning element by the user during use.

The processing arrangement may be adapted to determine the baseline or average loading force applied to the at least one cleaning element during a cleaning session, using the sensing signal from the sensing means. This may be done in some examples as part of a calibration operation.

To explain further, proposed embodiments involve receiving a sensing signal indicative of force, pressure or motion during use of the oral care device in a cleaning session. In such a context, the user applies a load to the cleaning elements 14 and the support body 12 as they clean their teeth. To increase the specificity of the measurements, the at least one further characteristic may include a user baseline load parameter, indicative of a baseline or average loading force exerted on the cleaning element by the user during use.

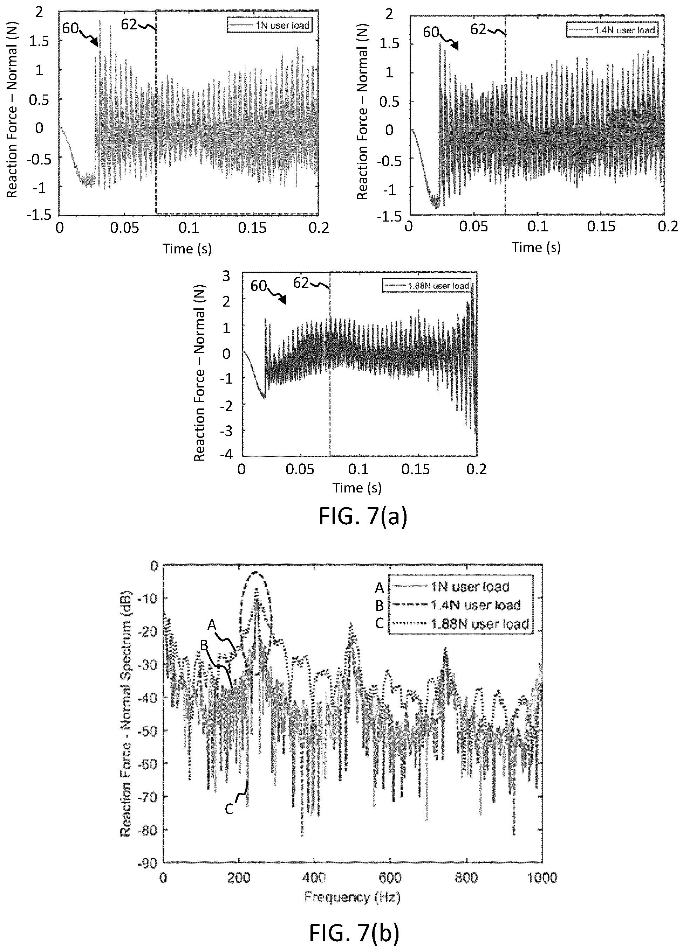

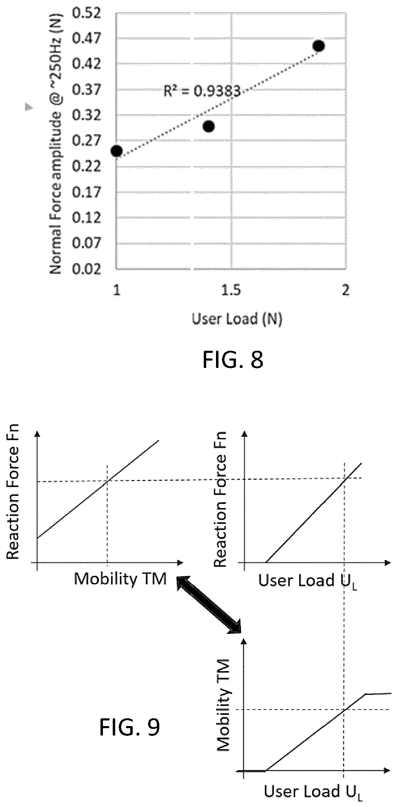

With reference to Fig. 7 and Fig. 8, with a new set of simulations, the inventors studied the impact of different applied user loads (1.88 N, 1.44 N and 1 N) on a measured reaction force on the support body 12, each for a simulated tooth with a same level of tooth mobility.

It was observed that the normal force amplitude at the main harmonic component of the driving oscillation applied to the cleaning elements had a monotonic, and approximately linear relationship with the user load ($U_L$).

Fig. 7(a) shows a waveform of the obtained normal reaction force sensing signal 60 for each of three different applied user loads (1N, 1.4N and 1.88N).

Fig. 7(b) shows a frequency spectrum computed for the signal segment 62 corresponding to a time window of 0.075s - 0.2s. This could be computed with a Fourier transform for example. The frequency spectra for each of the three different applied user loads are labelled A, B, C, with A corresponding to 1N load, B corresponding to 1.4N load, and C corresponding to 1.88N load.

Fig. 8 shows a derived approximately linear relationship between the normal force amplitude at the dominant driven oscillation frequency components (e.g. 250 Hz in the simulated example), and the applied user load.

It is noted that a similar linear relation between amplitude of tooth deflection and applied loading force i.e. $U_L$, has been reported in the literature. Reference is made for example to the paper: M. Goellner, J. Schmitt, S. Holst, A. Petschelt, M. Wichmann, and C. Berthold, "Correlations between tooth mobility and the Periotest method in periodontally involved teeth.," Quintessence Int, vol. 44, no. 4, pp. 307-316, Apr. 2013.

Thus, the sensing signal, e.g. sensed reaction force at the support body 12, is a function of tooth mobility; and the reaction force is in turn a function of user load ($U_L$). Therefore, as schematically illustrated in Fig. 9, tooth mobility (TM) can be also expressed as function of user load ($U_L$).

It is further noted that the user load is functionally related in some part to the structural properties of the cleaning elements 14 since the cleaning elements behave differently under different applied user loads. With varying load, the bending state of cleaning elements vary, and this affects a stiffness of the force transfer between the mobile tooth and the integrated sensor means via the cleaning elements.

Under low user load, the force transfer properties of the cleaning elements become less stiff and less sensitive to the tooth displacement. Therefore, there may exist a minimum user load, above which the dynamic behavior of the wobbling tooth can be sensed.

It has been found that use of user load alone as the further characteristic utilized in the tooth mobility computation is sufficient to provide an objective tooth mobility metric enabling meaningful comparison between mobility measurements at different time points.

In practice, a level of an applied user load could be sensed in a dedicated calibration operation, or could be computed based on the sensing signal acquired in one or more normal cleaning sessions. The user load could be detected as the baseline of the sensed force or pressure signal from the sensor means 30, wherein the applied user load can be expected to be the dominant component of the baseline, and thus relative changes in the baseline map onto changes in the user load. An average of user load could be obtained over multiple cleaning sessions in some examples.

With regards to determining the particular relationship between the user load and the sensor signal response for a given tooth mobility level, this could be based on a pre-determined (population average) relationship. Alternatively, a custom relationship could be derived for the specific user. For example, this could be achieved in a dedicated calibration procedure. In other words, in some embodiments, the processing arrangement 20 may be adapted to perform a calibration operation comprising sensing a baseline or average loading force applied to the at least one cleaning element during a cleaning session, and wherein the degree of tooth mobility is determined in dependence upon the baseline or average loading force.

For example, the user might be instructed to apply the cleaning elements 14 to a single tooth and to vary the load that they apply. The resulting measurement signal for the calibration procedure corresponds to a single tooth with a single level of mobility. The variations in the load being applied by the user can be detected for example by extracting the baseline of a force or pressure sensing signal, wherein the applied user load can be expected to be the dominant component of the baseline, and thus relative changes in the baseline map onto changes in the user load. At the same time, from the sensing signal for the calibration operation period, the amplitude of the dominant harmonic component of oscillation can be monitored as a function of time over this period. Then, the extracted user load signal and extracted amplitude signal can be temporarily registered to one another, to thereby derive a plot of the amplitude as a function of the (relative) applied user load, and from this a linear correlation between the two (i.e. a linear mapping) derived.

This correlation can then be applied in operation to calibrate the sensing signal according to varying user load during a cleaning session, or according to an average user load derived from data acquired over one or more cleaning sessions.

Another factor which affects the relationship between the sensing signal and the tooth mobility is the morphology of the tooth being cleaned. It has been found that single rooted and multi-rooted teeth can have significantly different mobility patterns. For example, the maximum mobility amplitude for molars may typically be around 40 $\mu$m, while the maximum mobility amplitude for incisors may typically be around 120 $\mu$m. See for example, M. Perlitsh, "A systematic approach to the interpretation of tooth mobility and its clinical implications," Dental clinics of North America, vol. 24, pp. 177-93, May 1980.

Therefore, according to some embodiments, morphological features of a tooth may be taken into account in computing the tooth mobility metric.

As discussed above, an indication of a tooth type can be obtained based on obtaining an identification of a tooth, where this could be derived based on a location sensor integrated in the device or based on analysis of the sensing signal waveform itself, or based on additional sensing components such as a camera integrated in the device and arranged to capture image data of the tooth being cleaned. Standard image processing methods can be used to determine a tooth type from the image data.

For each type of tooth, its mechanical properties may be determined in advance and a functional parameter stored in a memory of the processing arrangement for use in computing the mobility metric. For example, for each of a plurality of different possible tooth types, an effective tooth stiffness parameter, $k$, could be derived in advance, e.g. from experimental or population-based data. A value of $k$ for each tooth type could be stored in the memory. In operation, once the tooth type is detected, the corresponding stiffness parameter is retrieved. The tooth mobility metric computation may involve application of a function which includes k as an independent parameter/variable.

Additionally or alternatively, the at least one further characteristic may comprise one or more parameters relating to structural or mechanical characteristics of the at least one cleaning element.

In particular, the properties of the cleaning elements and support body (e.g. brush head, $b_H$) such as cleaning element stiffness, degree of cleaning element wear, and an angle of protrusion of the cleaning elements from the support body (or brushing angle ($b_A$)), are relevant parameters.

In particular, the angle of protrusion of the cleaning elements, and a metric of stiffness of the cleaning elements, are each fixed parameters of at least a portion of the oral care device, and can therefore be readily identified. In some examples, the support body and cleaning elements may be part of a cleaning unit (e.g. brush head) which is removably coupled to a main body of the device, to enable replacement. In some examples, an electronically readable tag (e.g. RFID tag) may be integrated in the cleaning unit part, readable by a sensor in the main body part, and the tag containing information about the structural characteristics of the cleaning elements and/or the support body. In some examples, the information could instead be input by a user (e.g. via an app loaded on a secondary device which is communicatively coupled with the processing arrangement).

In some embodiments, the at least one further characteristic may comprise a brushing stroke speed parameter indicative of a speed of user-applied brushing strokes during the cleaning operation.

This could be determined in a calibration operation and stored. It could be determined dynamically during the cleaning sessions, for instance using an IMU integrated in the device.

Thus, physical properties specific to the cleaning unit support body and cleaning elements (such as type of cleaning elements, stiffness of cleaning elements, frictional coefficient of cleaning elements, wear/deformation of cleaning elements, cleaning element layout) can be included among the at least one further characteristic.

In a preferred set of embodiments, the tooth mobility metric is derived using a combination of a plurality of the different further characteristics mentioned above. This can be achieved for example by defining a tooth mobility metric computation function, which is a function of multiple different input parameters. For example, It may be a linear function of the different further characteristics. The linear function may be derived using a multi-parametric linear regression technique, in which the coefficients of the linear function are derived in advance through a regression fitting operation based on experimental data. In the final device, the tooth mobility metric computation function may be stored on a memory of the processing arrangement and retrieved during operation for computation of the tooth mobility metric based on the measured waveform characteristic and based on the at least one further characteristic.

In some examples a machine learning model may be used to compute the metric of tooth mobility.

In some examples, a multi-parametric linear regression model may be used to compute the metric of tooth mobility.

To explain further, in general terms, a computation function, *f*, may be determined in advance, which is defined to provide a mapping from

a) the signal waveform characteristic derived from the sensing signal indicative of measured force applied to, or motion of, at least one cleaning element, and
b) the at least one further characteristic of the tooth, and/or of the mechanical engagement of the at least one cleaning element with the tooth
to
c) a metric of tooth mobility.

In the particular example discussed above, the signal waveform characteristic derived from the sensing signal is $F_n$, where $F_n$ in this context refers to the reaction force amplitude at the central frequency of the dominant peak in the frequency spectrum (i.e. force amplitude at the main harmonic or resonant frequency of oscillation).

With regards to (b), examples of the further characteristics mentioned above include: k = a stiffness metric of the tooth, $U_L$ = the user baseline load, $b_H$ = one or more structural properties of the cleaning elements such as stiffness and/or level of wear, and $b_A$ = the angle of the cleaning elements relative to the tooth surface during cleaning of a tooth (i.e. brushing angle).

Thus, deriving the tooth mobility metric, TM, may be performed based on the processing arrangement computing

$$TM = f(F_n,\ k,\ U_L,\ b_H,\ b_A)$$

where *f* is a pre-determined computation function as discussed above. As discussed above, the tooth mobility metric can be based on a change in $F_n$ from a reference frequency spectrum. The function *f* may be configured based on the reference frequency spectrum so as to provide an appropriate mapping to TM.

Of course, the above is just an example, and *f* may instead be a function of a different combination of one or more of the further characteristics of the tooth, and/or of the mechanical engagement of the at least one cleaning element with the tooth. Also, instead of $F_n$, a different characteristic of the sensing signal waveform may be used.

Once a standardized metric of tooth mobility has been obtained, in some embodiments this may be further processed to derive a clinical classification of the tooth mobility for the at least one tooth.

Thus, this set of embodiments proposes to classify the tooth mobility metric according to a clinically relevant category, allowing for clinical interpretation and cross-correlation between teeth and between cleaning sessions, to clinically assess tooth mobility.

Deriving the clinical classification may comprise comparing the obtained tooth mobility metric with a selected one or more thresholds.

Optionally, the one or more thresholds to which the tooth mobility metric is compared may be selected based on at least one of: a type of tooth, and a demographic classification of the user.

For example, during a brushing session, a sensing signal is received from the sensing means (e.g. reaction force measurement). This includes signal segments corresponding to a series of different dental locations, each spanning a particular time window of the signal. A particular tooth to which each signal segment corresponds can be identified, as discussed above, based on localization methods such as using position or orientation sensor means integrated in the device, or any other suitable technique.

The measured sensing signal segment 62 for each tooth can then be processed in combination with one or more of the further characteristics mentioned above, to compute the tooth mobility metric for the corresponding tooth, using for instance a linear regression model or other suitable equation, function or algorithm.

To derive the clinical classification for each tooth, the derived TM values may be compared with a pre-defined threshold specific to the particular tooth. In this way, teeth may be identified which exhibit pathological mobility. In some examples, a graded classification indicative of a level of pathology may be determined.

The classification may be one of a set of discrete classifications, e.g. no disease, low level disease, moderate disease, severe disease. The discrete classification may be derived by comparing the derived tooth mobility metric with one or more thresholds specific to the tooth. In some examples, the classification may be a graded score along a pathology scale, e.g. +1 to +10.

As different types of teeth have different normal levels of mobility, a lookup table may be used storing appropriate tooth mobility thresholds for the different classifications, for each of a plurality of different tooth types. These thresholds could be derived from literature or could be derived empirically using experimental data.

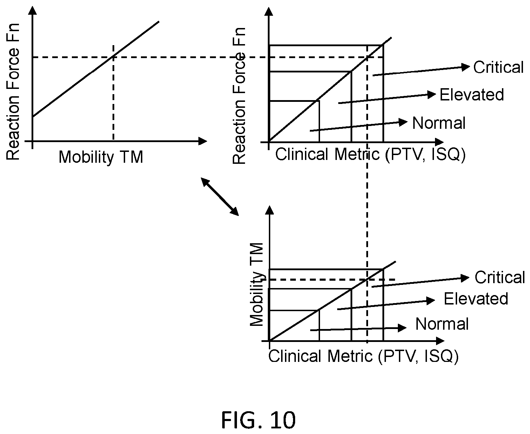

Fig. 10 schematically illustrates how pre-determined tooth mobility thresholds could be applied to derive a clinical classification.

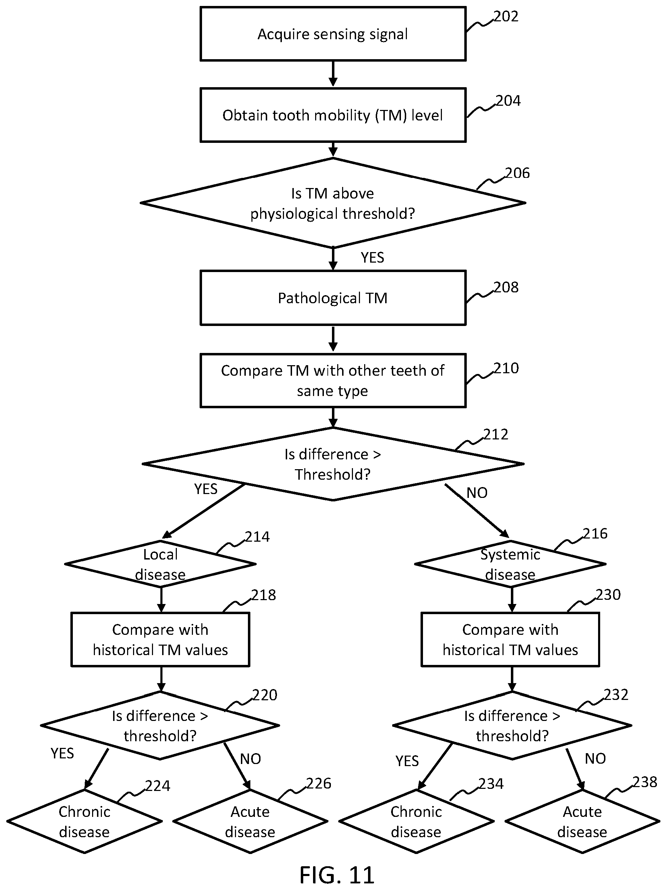

Fig. 11 outlines in block diagram form a decision flow for deriving a clinical classification.

The sensing signal is acquired 202 over the course of the cleaning session, and a tooth mobility metric derived 204 for each tooth, in accordance with procedures outlined above.

The tooth mobility metric is compared 206 with a physiological threshold, which may be specific to the particular tooth, as discussed above.

If the tooth mobility is above the physiological threshold, then a conclusion is reached 208 that the tooth mobility is pathological (i.e. abnormal and indicative of some form of disease). If not, then a conclusion may be reached that the tooth mobility is non-pathological and an indication of this may be output in the form of a data signal.

A first stage in deriving a clinical classification for the tooth mobility for the given tooth may then comprise comparing 210 the tooth mobility metric obtained for the tooth with that of a further tooth to detect a level of deviation between the two. The further tooth is preferably a tooth of a same tooth type, and on an opposite side of the dental arch to the first tooth (e.g. right upper first molar is compared with left upper first molar). Additionally or alternatively, tooth mobility metrics may be obtained for multiple segments of each tooth, and the metrics for matching segments on opposite sides of the mouth compared.

The level of deviation between the two is then compared 212 with a deviation threshold.

If the level of deviation exceeds the threshold, then the disease may be classified as local disease 214. If the level of deviation does not exceed the threshold, the level of disease may be classified as systemic disease 216.

The tooth mobility metric derived for each tooth (or tooth segment) may then further be compared 218, 230 with previous tooth mobility metric values derived for the same tooth or tooth segment, to see if there has been deterioration. For example, the method may comprise accessing a datastore recording historical tooth mobility metric data for a user; comparing the obtained tooth mobility metric for the at least one tooth with historical values for the same tooth and wherein the clinical classification is further based on said comparison.

If the difference between the current tooth mobility metric for the given tooth and the previous value(s) is greater than a predetermined threshold 220, 232, the disease may be classified as a chronic disease 224, 234; if not then the disease may be classified as acute disease 226, 238. In alternative examples, the reverse logic might instead be applied: persistently high tooth mobility may indicate chronic disease, whereas a sudden increase may indicate acute disease.

In particular, a significant deviation in the tooth mobility metric values indicates for example progressive disease. This information is then communicated to the user or to a dental practitioner, for example using a user interface component.

In some embodiments, the processing arrangement may be further adapted to generate a warning and/or a recommendation to schedule a visit to a dental practitioner in dependence upon the derived clinical classification. In some examples, this may be dependent on a trend in the tooth mobility values or the clinical classification, e.g. a deterioration in mobility or disease.

Optionally, the derived tooth mobility metric values, and/or clinical classifications, for each tooth may be stored in a memory for future reference.

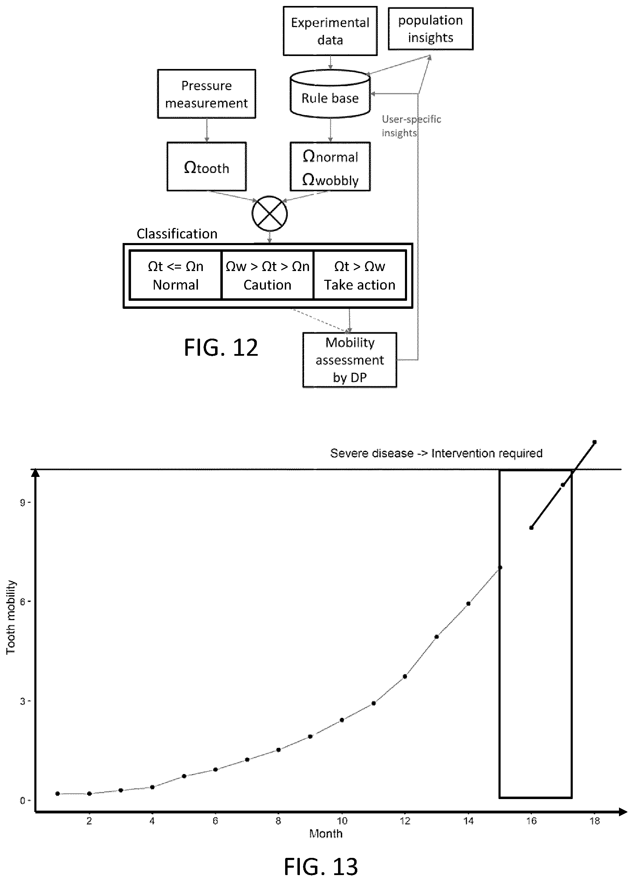

By way of further illustration, Fig. 12 illustrates the logical architecture for deriving a clinical classification based on tooth mobility. As illustrated, it is proposed to derive a measure of tooth mobility for one or more teeth (represented by $\Omega$tooth in Fig. 12), where this is derived from sensor data from the oral care device, e.g. from force or pressure measurements. In addition, it is proposed to create in advance a rule base stored in a datastore which records a set of thresholds against which the derived measure of tooth mobility for the user is compared to obtain a clinical classification. The thresholds in the illustrated example include $\Omega$normal and Qwobbly. As illustrated in Fig. 12, in this example, if $\Omega$tooth <= $\Omega$normal, then no action is required. If Qwobbly > $\Omega$tooth > $\Omega$normal, then the clinical classification may be an early warning classification, and the user may be advised to remain vigilant for progression of tooth mobility. If $\Omega$tooth > Qwobbly, then the clinical classification may be that the tooth has abnormal mobility and the user may be advised to seek advice from a dental practitioner.

With regards to the thresholds stored in the rule base, these may be derived based on experimental data, e.g. a population database containing tooth mobility measurements and corresponding diagnoses/classifications from a dental practitioner. Thus, the thresholds may be set based on a combination of experimental data (tooth mobility measurements) and data from a dental practitioner. The thresholds furthermore may be personalized to the user, based on past mobility measurements for the user, and/or based on input from a dental practitioner specific to the user, e.g. the dental practitioner adjusts or sets the thresholds based on physical examination of the user.

According to some embodiments, the processing arrangement may be adapted to derive a prediction of tooth mobility progression, for example based on use of a machine learning algorithm trained on a database of historical data for a population of users.

For example, in at least one set of embodiments, the machine learning algorithm may be trained to receive as inputs: the derived tooth mobility metric for a single cleaning session, and a demographic classification of the user, and to generate as an output a prediction of tooth mobility progression.

This machine learning algorithm may be trained in advance based on training data for a population of multiple patients in different demographic classifications, the training data comprising, for each of the multiple patients, tooth mobility metrics for at least two different time points, and a demographic classification for the patient. The training data may be data extracted from a population database.

Additionally or alternatively, a machine learning model may be used which is trained to receive as inputs: the derived tooth mobility metric for a series of cleaning sessions for a series of time points, and to generate as an output a prediction of tooth mobility progression, and wherein the algorithm has been trained based on training data for a population of multiple patients, the training data comprising for each of the multiple patients tooth mobility metrics for a series of different time points. Again, the training data may be data extracted from a population database.

For example, a population database might be utilized containing recorded sensing signal data for a plurality of cleaning sessions for a plurality of users, and also recording other parameters related to the cleaning elements or the mechanical engagement of the cleaning elements with the teeth, e.g. brushing angle, tooth type etc. The database also records the corresponding tooth mobility measurements and clinical classifications for each tooth included in the sensing signal data for each user. The data can be longitudinal, i.e. for each individual it contains data over a prolonged period of time. Also, other data can be included, such as demographic data, dental data, and data related to brushing behavior for each user, which enables tailored prediction (i.e. based on group levels).

To explain further, the population database may contain relevant data for a large number of individuals. This may include, for each individual, longitudinal data for tooth mobility over an extended period of time (e.g. from first onset of mobility onwards). It may also include other data for each patient, such as demographic data.

The data acquired for the user of the device may comprise only very limited longitudinal data on tooth mobility. For example, it may comprise tooth mobility data covering only a relatively short amount of time.

The processing arrangement 20 may be adapted to use the population database to generate a prediction for the user as to future progression of tooth mobility over time.

To implement this, a machine learning algorithm can be applied, where the machine learning algorithm has been trained based on the population database.

According to a further example, a 'nearest neighbor approach' may instead be applied to generate a prediction. For example, a subset of the population database may be selected based on a computed similarity metric between the data of the selected subset and the data for the current user: e.g. a similarity between the user's longitudinal mobility data, and demographic factors, with longitudinal mobility data and demographic data of the population database subset. A prediction of future progression of tooth mobility for the user may then be derived based on the progression of tooth mobility for individuals in the selected subset of the population database.

As different teeth have different mobility profiles, different machine learning models could trained in advance and applied for different tooth types. Furthermore, different machine learning models could be trained and used for different types or classifications of disease. The type or classification of disease (e.g. acute vs chronic; local vs systemic) has a bearing on tooth mobility progression, and therefore for different diseases models could be modified, or weights adapted.

Use of a machine learning model for prediction represents just one example. Other types of algorithm, function or model could alternatively be used.

In algorithmic form, tooth mobility prediction can be considered as time series forecasting, where future tooth mobility values are predicted based on previously observed values. Various mathematical models, such as moving average, or Autoregressive integrated moving average (ARIMA) could be used for this purpose.

Fig. 13 illustrates an example result of tooth mobility prediction using an ARIMA model, based on previous tooth mobility values. The previous values are those within the light grey region. The predicted values, forming a forward-projected trend, are those in the darker grey region. A severe disease threshold line is marked on the chart. It can be seen that the predicted future trend enables a predicted future time at which the tooth mobility values will exceed the threshold.

If the predicted value of tooth mobility is beyond a certain predefined threshold, this could be indicated to a user, for example by generating a sensory output, e.g. by audio-visual means. This information can be used by a user for example for planning a visit to DP's office.

Optionally, the population database may include data for both users that have received mobility treatment and those that haven't. This could potentially enable generation of two predictions (the forecast of both scenarios: with and without TM treatment) and thereby these insights can be used for motivating the user towards treatment.

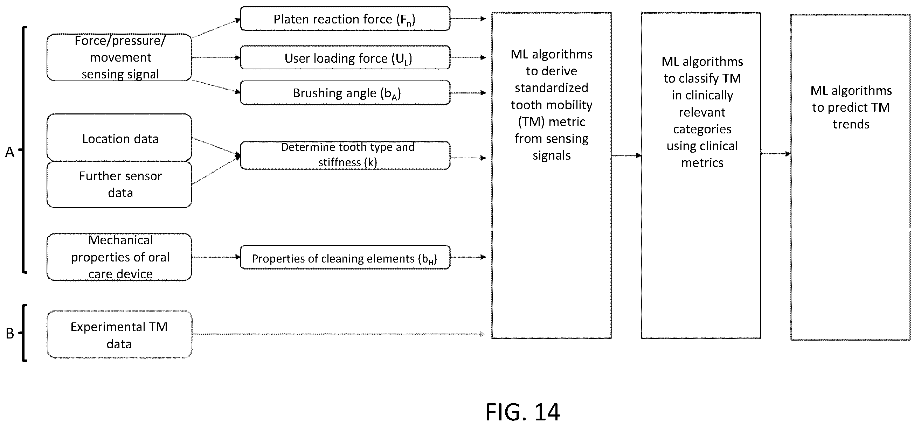

By way of summary a flowchart outlining the various features which may be implemented in accordance with different embodiments of the invention is shown in in Fig. 14.

The parameters shown in region B may be required only during training of the algorithms used in computing the different parameters. Although this example chart focusses on parameters for a toothbrush, the same principles can be applied to another type of oral care device, such as a brushing mouthpiece device.

In summary, force, pressure, or motion sensing data, in addition to at least one further parameter related to a tooth or to tooth-device engagement properties, are used as inputs to derive a tooth mobility (TM) metric. As mentioned in Fig. 14, this could be based on application of a machine learning (ML) model, trained using experimental or population TM data. The derived tooth mobility metric may then be used to clinically classify the mobility of each tooth, based on one or more thresholds. Predictions of future tooth mobility trends for one or more teeth may further be derived.

Although Fig. 14 refers by way of illustration to machine learning (ML) algorithms, any other suitable type of algorithm or function could instead be used.

Embodiments of the invention described above employ a processing arrangement. The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims, the scope of the invention being defined by the wording of the claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A processing arrangement (20) for an oral care device (10), comprising:

   an input/output (22); and
   one or more processors (24), adapted to:

   receive at the input/output (22) a sensing signal (32) indicative of a loading force applied to, or motion of, at least one cleaning element (14) of the oral care device, wherein the signal spans a time period, the time period corresponding to a period of mechanical engagement of the at least one cleaning element with at least one tooth (40) during an oral cleaning session,
   process a waveform (60) of the signal to extract one or more characteristics of the signal waveform for the time period;
   obtain a measure of at least one further characteristic, the further characteristic being the tooth type, and/or a characteristic of the mechanical engagement of the at least one cleaning element with the tooth;
   determine a metric of tooth mobility (26) for the at least one tooth in dependence of the one or more signal characteristics and upon the at least one further characteristic; and
   generate a data signal (25) indicative of the determined metric, and provide the data signal to the input/output.

2. The processing arrangement (20) of claim 1, wherein the metric of tooth mobility (26) defines a graded level of tooth mobility.

3. The processing arrangement of claim 1 or 2, the processing arrangement further adapted to:

   access a datastore (28) recording historical tooth mobility metric data for a user; and
   output the data signal (25) to the datastore, for thereby storing the determined tooth mobility metric in the datastore.

4. The processing arrangement (20) of claim 3, wherein:

   the one or more processors are adapted to access the datastore (28) recording historical tooth mobility metric data for the user;
   the one or more processors are further adapted to determine a longitudinal trend in tooth mobility metric for one or more teeth of the user based on the historical tooth mobility metric data and the determined metric of tooth mobility; and
   preferably wherein the one or more processors are adapted to generate a prediction of tooth mobility progression based on the longitudinal trend.

5. The processing arrangement of any of claims 1-4, the processing arrangement further adapted to obtain an identification of the at least one tooth, and wherein the data signal further includes the identification of the at least one tooth.

6. The processing arrangement of claim 5, wherein the received sensing signal comprises signal segments (62) corresponding to respective periods of engagement of the at least one cleaning element with each of a plurality of teeth, and the processing arrangement adapted to determine a respective metric of tooth mobility for each of the plurality of teeth, and obtain an identification of each of the teeth.

7. The processing arrangement of claim 6, further comprising identifying a start and end of each signal segment by:

   detecting a pre-determined signature pattern indicative of start and end of each tooth segment; or
   using of a positioning signal for the at least one cleaning element, received from a positioning signal source.

8. The processing arrangement of any of claims 1-7, wherein the at least one further characteristic comprises a user baseline load parameter, indicative of a baseline or average loading force exerted on the cleaning element by the user during use, and
   optionally wherein the processing arrangement is adapted to perform a calibration operation comprising determining the baseline or average loading force applied to the at least one cleaning element during a cleaning session, using the sensing signal.

9. The processing arrangement of any of claims 1-8, wherein the at least one further characteristics comprises:

   a brushing stroke speed parameter indicative of a speed of user-applied brushing strokes during the cleaning operation; and/or
   one or more parameters relating to structural characteristics of the at least one cleaning element, and wherein these include one or more of:

   - an angle of protrusion of the cleaning elements; and
   - a metric of stiffness of the cleaning elements.

10. The processing arrangement of any of claims 1-9, further adapted to process the obtained tooth mobility metric and to derive a clinical classification of the tooth mobility for the at least one tooth.

11. The processing arrangement of claim 10, wherein deriving the clinical classification comprises comparing the obtained tooth mobility metric with a selected one or more thresholds, and optionally wherein the one or more thresholds are selected based on at least one of: a type of tooth, and a demographic classification of the user.

12. The processing arrangement of claim 10 or 11,

   wherein the sensing signal includes signal segments corresponding to periods of engagement of the at least one cleaning element with a plurality of teeth, and wherein the processor is adapted to determine a respective metric of tooth mobility for each of the plurality of teeth; and

wherein deriving the clinical classification for the tooth mobility for a first tooth further comprises comparing the tooth mobility metric obtained for the first tooth with a further tooth to detect a level of deviation, wherein the further tooth is of a same tooth type, and on an opposite side of the dental arch to the first tooth, and comparing the level of deviation with a deviation threshold.

13. The processing arrangement of any of claims 10-12, wherein the processor is further adapted to:

access a datastore recording historical tooth mobility metric data for a user;

compare the obtained tooth mobility metric for the at least one tooth with historical values for the same tooth; and

wherein the clinical classification is further based on said comparison.

14. The processing arrangement of any of claims 1-13, wherein the processor is adapted to generate a prediction of tooth mobility progression based on application of a machine learning algorithm,

wherein the machine learning algorithm is trained to receive as inputs: the derived tooth mobility metric for a single cleaning session, and a demographic classification of the user, and to generate as an output a prediction of tooth mobility progression, and wherein the algorithm has been trained based on training data for a population of multiple patients in different demographic classifications, the training data comprising for each of the multiple patients tooth mobility metrics at two different time points, and a demographic classification for the patient.

15. An oral care device comprising:

a support body;

a plurality of cleaning elements for engagement with oral surfaces, the cleaning elements protruding from a surface of the support body;

a sensor means adapted to sense a measure indicative of a loading force or pressure applied to, or a motion of, at least one of the cleaning elements; and

a processing arrangement in accordance with any of claims 1-14.

**Patentansprüche**

1. Verarbeitungsanordnung (20) für eine Mundpflegevorrichtung (10), umfassend:

eine Eingabe/Ausgabe (22); und

einen oder mehrere Prozessoren (24), die ausgelegt sind, um:

bei der Eingabe/Ausgabe (22) ein Sensorsignal (32) zu empfangen, das auf eine auf mindestens ein Reinigungselement (14) der Mundpflegevorrichtung wirkende Belastungskraft oder Bewegung dieses Elements hinweist, wobei sich das Signal über einen Zeitraum erstreckt, der

einem Zeitraum eines mechanischen Eingriffs des mindestens einen Reinigungselements mit mindestens einem Zahn (40) während einer Mundreinigungssitzung entspricht,

eine Wellenform (60) des Signals zu verarbeiten, um eine oder mehrere Charakteristika der Signalwellenform für den Zeitraum zu extrahieren;

ein Maß für mindestens eine weitere Charakteristik zu erhalten, wobei es sich bei der weiteren Charakteristik um den Zahntyp und/oder eine Charakteristik des mechanischen Eingriffs des mindestens einen Reinigungselements mit dem Zahn handelt;

eine Zahnbeweglichkeitsmetrik (26) des mindestens einen Zahns in Abhängigkeit von der einen oder den mehreren Signalcharakteristika und mindestens einer weiteren Charakteristik zu bestimmen; und ein Datensignal (25) zu generieren, das die bestimmte Metrik anzeigt, und das Datensignal der Eingabe/Ausgabe bereitzustellen.

2. Verarbeitungsanordnung (20) nach Anspruch 1, wobei die Zahnbeweglichkeitsmetrik (26) einen abgestuften Grad an Zahnbeweglichkeit definiert.

3. Verarbeitungsanordnung nach Anspruch 1 oder 2, wobei die Verarbeitungsanordnung weiter ausgelegt ist, um:

auf einen Datenspeicher (28) zuzugreifen, der Verlaufsdaten zur Zahnbeweglichkeitsmetrik eines Benutzers aufzeichnet; und

das Datensignal (25) an den Datenspeicher auszugeben, um so die bestimmte Zahnbeweglichkeitsmetrik im Datenspeicher zu speichern.

4. Verarbeitungsanordnung (20) nach Anspruch 3, wobei:

der oder die Prozessoren ausgelegt sind, um auf den Datenspeicher (28) zuzugreifen, der Verlaufsdaten zur Zahnbeweglichkeitsmetrik des Benutzers aufzeichnet;

der oder die Prozessoren weiter ausgelegt sind, um einen Längsschnitt-Trend der Zahnbeweglichkeitsmetrik für einen Zahn oder mehrere Zähne des Benutzers auf der Grundlage der Verlaufsdaten zur Zahnbeweglichkeitsmetrik und der bestimmten Zahnbeweglichkeitsmetrik zu bestimmen; und

wobei bevorzugt der eine oder die mehreren Prozessoren ausgelegt sind, um eine Vorhersage über das Fortschreiten der Zahnbeweglichkeit auf der Grundlage des Längsschnitt-Trends zu generieren.

5. Verarbeitungsanordnung nach einem der Ansprüche 1-4, wobei die Verarbeitungsanordnung weiter ausgelegt ist, um eine Identifizierung des mindestens einen Zahns zu erhalten, und wobei das Datensignal weiter die Identifizierung des mindestens einen Zahns beinhaltet.

6. Verarbeitungsanordnung nach Anspruch 5, wobei das empfangene Sensorsignal Signalsegmente (62) umfasst, die jeweils den Eingriffsperioden des mindestens einen Reinigungselements mit jedem der mehreren Zähne entsprechen, und die Verarbeitungsanordnung ausgelegt ist, um für jeden der Zähne eine jeweilige Zahnbeweglichkeitsmetrik zu bestimmen und eine Identifizierung jedes einzelnen Zahns zu erhalten.

7. Verarbeitungsanordnung nach Anspruch 6, weiter umfassend das Identifizieren eines Anfangs und eines Endes jedes Signalsegments durch:

Detektieren eines vorbestimmten Signaturmusters, das den Anfang und das Ende jedes Zahnsegments anzeigt; oder

Verwenden eines Positionierungssignals für das mindestens eine Reinigungselement, das von einer Positionierungssignalquelle empfangen wird.

8. Verarbeitungsvorrichtung nach einem der Ansprüche 1-7, wobei das mindestens eine weitere Charakteristik einen Benutzer-Basislastparameter umfasst, der eine Basis- oder durchschnittliche Belastungskraft angibt, die der Benutzer während der Benutzung auf das Reinigungselement ausübt, und

wobei optional die Verarbeitungsanordnung ausgelegt ist, um einen Kalibrierungsvorgang durchzuführen, der Bestimmen der Basis- oder durchschnittlichen Belastungskraft, die während eines Reinigungsvorgangs auf das mindestens eine Reinigungselement ausgeübt wird, unter Verwendung des Sensorsignals umfasst.

9. Verarbeitungsanordnung nach einem der Ansprüche 1-8, wobei die mindestens eine weitere Charakteristik Folgendes umfasst:

einen Parameter für die Bürstenstrichgeschwindigkeit, der die Geschwindigkeit der vom Benutzer während des Reinigungsvorgangs ausgeführten Bürstenstriche angibt; und/oder

einen oder mehrere Parameter, die sich auf die strukturellen Charakteristika des mindestens einen Reinigungselements beziehen, wobei diese eines oder mehrere der Folgenden umfassen:

- einen Austrittswinkel der Reinigungselemente; und
- eine Metrik für die Steifigkeit der Reinigungselemente.

10. Verarbeitungsanordnung nach einem der Ansprüche 1-9, die weiter ausgelegt ist, um die erhaltene Zahnbeweglichkeitsmetrik zu verarbeiten und eine klinische Klassifizierung der Zahnbeweglichkeit für mindestens einen Zahn abzuleiten.

11. Verarbeitungsanordnung nach Anspruch 10, wobei das Ableiten der klinischen Klassifizierung das Vergleichen der erhaltenen Zahnbeweglichkeitsmetrik mit einem oder mehreren ausgewählten Schwellenwerten umfasst, wobei der oder die Schwellenwerte optional auf der Grundlage mindestens eines der folgenden Kriterien ausgewählt werden: einem Zahntyp und einer demografischen Klassifizierung des Benutzers.

**12.** Verarbeitungsanordnung nach Anspruch 10 oder 11,

wobei das Sensorsignal Signalsegmente umfasst, die den Eingriffszeiten des mindestens einen Reinigungselements mit mehreren Zähnen entsprechen, und wobei der Prozessor ausgelegt ist, um für jeden der Zähne eine entsprechende Zahnbeweglichkeitsmetrik zu bestimmen; und

wobei Ableiten der klinischen Klassifizierung für die Zahnbeweglichkeit eines ersten Zahns weiter das Vergleichen der für den ersten Zahn erhaltenen Zahnbeweglichkeitsmetrik mit einem weiteren Zahn umfasst, um einen Grad der Abweichung zu detektieren, wobei der weitere Zahn vom gleichen Zahntyp ist und sich auf der gegenüberliegenden Seite des Zahnbogens zum ersten Zahn befindet, sowie Vergleichen des Grades an Abweichung mit einem Abweichungsschwellenwert.

**13.** Verarbeitungsanordnung nach einem der Ansprüche 10-12, wobei der Prozessor weiter ausgelegt ist, um:

auf einen Datenspeicher zuzugreifen, der Verlaufsdaten zur Zahnbeweglichkeitsmetrik eines Benutzers aufzeichnet;

die erhaltene Zahnbeweglichkeitsmetrik für mindestens einen Zahn mit historischen Werten für denselben Zahn zu vergleichen; und

wobei die klinische Klassifizierung weiter auf diesem Vergleich basiert.

**14.** Verarbeitungsvorrichtung nach einem der Ansprüche 1-13, wobei der Prozessor ausgelegt ist, um eine Vorhersage über einen Fortschritt der Zahnbeweglichkeit auf der Grundlage der Anwendung eines Algorithmus für maschinelles Lernen zu generieren,

wobei der Algorithmus für maschinelles Lernen darauf trainiert wird, als Eingaben die abgeleitete Zahnbeweglichkeitsmetrik für eine einzelne Reinigungssitzung und eine demografische Klassifizierung des Benutzers zu empfangen und als Ausgabe eine Vorhersage über das Fortschreiten der Zahnbeweglichkeit zu generieren, und wobei der Algorithmus auf der Grundlage von Trainingsdaten für eine Population von mehreren Patienten in verschiedenen demografischen Klassifizierungen trainiert wurde, wobei die Trainingsdaten für jeden der mehreren Patienten Zahnbeweglichkeitsmetriken zu zwei verschiedenen Zeitpunkten und eine demografische Klassifizierung für den Patienten umfassen.

**15.** Mundpflegevorrichtung, umfassend:

einen Trägerkörper;

mehrere Reinigungselemente für einen Eingriff mit Mundoberflächen, wobei die Reinigungselemente aus einer Oberfläche des Trägerkörpers herausragen;

einen Sensor, der ausgelegt ist, um ein Maß zu erfassen, das auf eine auf mindestens eines der Reinigungselemente wirkende Belastungskraft oder einen Druck oder auf eine Bewegung dieses Elements hinweist; und

eine Verarbeitungsanordnung nach einem der Ansprüche 1-14.

**Revendications**

**1.** Agencement de traitement (20) pour un dispositif d'hygiène buccale (10), comprenant :

une entrée/sortie (22) ; et

un ou plusieurs processeurs (24), adaptés pour :

recevoir au niveau de l'entrée/sortie (22) un signal de détection (32) indiquant une force de chargement appliquée à, ou un mouvement de, au moins un élément de nettoyage (14) du dispositif d'hygiène buccale, dans lequel le signal couvre une période de temps, la période de temps correspondant à une période de mise en prise mécanique du au moins un élément de nettoyage avec au moins une dent (40) pendant une séance de nettoyage buccal,

traiter une forme d'onde (60) du signal afin d'extraire une ou plusieurs caractéristiques de la forme d'onde de signal pendant la période de temps ;

obtenir une mesure d'au moins une caractéristique supplémentaire, la caractéristique supplémentaire étant le type de dent et/ou une caractéristique de la mise en prise mécanique du au moins un élément de nettoyage avec la dent :

déterminer une mesure de la mobilité dentaire (26) pour la au moins une dent en fonction des une ou plusieurs caractéristiques de signal et de la au moins une caractéristique supplémentaire ; et

générer un signal de données (25) indiquant la mesure déterminée et fournir le signal de données à l'entrée/la sortie.

2. Agencement de traitement (20) selon la revendication 1, dans lequel la mesure de la mobilité dentaire (26) définit un degré gradué de mobilité dentaire.

3. Agencement de traitement selon la revendication 1 ou 2, l'agencement de traitement étant adapté en outre pour :

accéder à un magasin de données (28) enregistrant des données antérieures de mesure de mobilité dentaire pour un utilisateur ; et

transmettre le signal de données (25) au magasin de données, pour stocker ainsi la mesure de mobilité dentaire déterminée dans le magasin de données.

4. Agencement de traitement (20) selon la revendication 3, dans lequel :

les un ou plusieurs processeurs sont adaptés pour accéder au magasin de données (28) enregistrant des données antérieures de mesure de mobilité dentaire pour l'utilisateur ;

les un ou plusieurs processeurs sont adaptés en outre pour déterminer une tendance longitudinale de mesure de mobilité dentaire pour une ou plusieurs dents de l'utilisateur sur la base des données antérieures de mesure de mobilité dentaire et de la mesure déterminée de la mobilité dentaire ; et

de préférence dans lequel les un ou plusieurs processeurs sont adaptés pour générer une prédiction de la progression de mobilité dentaire sur la base de la tendance longitudinale.

5. Agencement de traitement selon l'une quelconque des revendications 1-4, l'agencement de traitement étant adapté en outre pour obtenir une identification de la au moins une dent, et dans lequel le signal de données inclut en outre l'identification de la au moins une dent.

6. Agencement de traitement selon la revendication 5, dans lequel le signal de détection reçu comprend des segments de signal (62) correspondant à des périodes respectives de mise en prise du au moins un élément de nettoyage avec chacune parmi une pluralité de dents, et l'agencement de traitement étant adapté pour déterminer une mesure respective de la mobilité dentaire pour chacune parmi la pluralité de dents et obtenir une identification de chacune des dents.

7. Agencement de traitement selon la revendication 6, comprenant en outre l'identification d'un début et d'une fin pour chaque segment de signal en :

détectant un motif de signature prédéterminé indiquant le début et la fin de chaque segment dentaire ; ou

utilisant un signal de positionnement pour le au moins un élément de nettoyage, reçu à partir d'une source de signal de positionnement.

8. Agencement de traitement selon l'une quelconque des revendications 1-7, dans lequel la au moins une caractéristique supplémentaire comprend un paramètre de charge initial utilisateur, indiquant une force de chargement initiale ou moyenne exercée sur l'élément de nettoyage par l'utilisateur pendant l'utilisation, et facultativement dans lequel l'agencement de traitement est adapté pour réaliser une opération d'étalonnage comprenant la détermination de la force de charge initiale ou moyenne appliquée au au moins un élément de nettoyage pendant une séance de nettoyage, en utilisant le signal de détection.

9. Agencement de traitement selon l'une quelconque des revendications 1-8, dans lequel la au moins une caractéristique supplémentaire comprend :

un paramètre de vitesse de course de brossage indiquant une vitesse de courses de brossage appliquées par l'utilisateur pendant l'opération de nettoyage ; et/ou

un ou plusieurs paramètres relatifs à des caractéristiques structurelles du au moins un élément de nettoyage, et dans lequel ceux-ci incluent un ou plusieurs éléments parmi :

- un angle de saillie des éléments de nettoyage ; et

- une mesure de rigidité des éléments de nettoyage.

10. Agencement de traitement selon l'une quelconque des revendications 1-9, adapté en outre pour traiter la mesure de mobilité dentaire obtenue et pour dériver une classification clinique de la mobilité dentaire pour la au moins une dent.

11. Agencement de traitement selon la revendication 10, dans lequel la dérivation de la classification clinique comprend la comparaison de la mesure de mobilité dentaire obtenue avec un ou plusieurs seuils sélectionnés, et facultativement dans lequel les un ou plusieurs seuils sont sélectionnés sur la base d'au moins un élément parmi : un type de dent et une classification démographique de l'utilisateur.

12. Agencement de traitement selon la revendication 10 ou 11,

dans lequel le signal de détection inclut des segments de signal correspondant à des périodes de mise en prise du au moins un élément de nettoyage avec une pluralité de dents, et dans lequel le processeur est adapté pour déterminer une mesure respective de la mobilité dentaire pour chacune parmi la pluralité de dents ; et
dans lequel la dérivation de la classification clinique pour la mobilité dentaire d'une première dent comprend en outre la comparaison de la mesure de mobilité dentaire obtenue pour la première dent avec une dent supplémentaire pour détecter un degré d'écart, dans lequel la dent supplémentaire est du même type de dent, et sur un côté opposé de l'arcade dentaire par rapport à la première dent, et la comparaison du degré d'écart avec un seuil d'écart.

13. Agencement de traitement selon l'une quelconque des revendications 10-12, dans lequel le processeur est adapté en outre pour :

accéder à un magasin de données enregistrant des données antérieures de mesure de mobilité dentaire pour un utilisateur ;
comparer la mesure de mobilité dentaire obtenue pour la au moins une dent avec des valeurs antérieures pour la même dent ; et
dans lequel la classification clinique est basée en outre sur ladite comparaison.

14. Agencement de traitement selon l'une quelconque des revendications 1-13, dans lequel le processeur est adapté pour générer une prédiction de progression de mobilité dentaire sur la base de l'application d'un algorithme d'apprentissage automatique,
dans lequel l'algorithme d'apprentissage automatique est entraîné pour recevoir comme entrées : la mesure de mobilité dentaire dérivée pour une seule séance de nettoyage, et une classification de données démographiques de l'utilisateur, et pour générer comme sortie une prédiction de progression de mobilité dentaire, et dans lequel l'algorithme a été entraîné sur la base de données d'entraînement pour une population de multiples patients appartenant à différentes classifications de données démographiques, les données d'entraînement comprenant pour chacun des multiples patients des mesures de mobilité dentaire à deux moments différents, et une classification de données démographiques pour le patient.

15. Dispositif d'hygiène buccale comprenant :

un corps de support ;
une pluralité d'éléments de nettoyage pour la mise en prise avec des surfaces buccales, les éléments de nettoyage faisant saillie depuis une surface du corps de support ;
un moyen de capteur adapté pour détecter une mesure indiquant une force de chargement ou une pression appliquée à, ou un mouvement de, au moins l'un des éléments de nettoyage ; et
un agencement de traitement selon l'une quelconque des revendications 1-14.

12
30
14

10

16

19

20
18

FIG. 1(a)

42 40
12 14

FIG. 1(b)

10

20
32
30

22
I/O
Force/
motion
Sensor
means

24
proc
25

Tooth mobility
metric
26

8

Datastore
28

Secondary Device
112

104
Proc

106
U/I

FIG. 2

FIG. 3

FIG. 4

FIG. 5(a)

FIG. 5(b)

FIG. 6(a)

FIG. 6(b)

FIG. 6(c)

FIG. 7(a)

FIG. 7(b)

FIG. 8

FIG. 9

FIG. 10

FIG. 11

FIG. 12

FIG. 13

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 112107386 A

**Non-patent literature cited in the description**

- **M. GOELLNER** ; **J. SCHMITT** ; **S. HOLST** ; **A. PETSCHELT** ; **M. WICHMANN** ; **C. BERTHOLD**. Correlations between tooth mobility and the Periotest method in periodontally involved teeth. *Quintessence Int*, April 2013, vol. 44 (4), 307-316

- **M. PERLITSH**. A systematic approach to the interpretation of tooth mobility and its clinical implications. *Dental clinics of North America*, May 1980, vol. 24, 177-93